# DEMANDE DE BREVET EUROPEEN

(11) **EP 0 695 546 A1**
(43) Date de publication de la demande: **07.02.1996**
(21) Numéro de dépôt: 95401290.2
(22) Date de dépôt: 02.06.1995
(51) Int. Cl.: A61K 31/165, A61K 31/615

(54) **Poudres à base de métoclopramide et de paracétamol ou de dérivés d'acide acétylsalicyclique**

(30) Priorité: 08.06.1994 FR 9407020
(71) Demandeur: SYNTHELABO, F-92350 Le Plessis Robinson (FR)
(72) Inventeur: Depauw, Thierry, F-37230 Fondettes (FR); Senequier, Raymond, F-37540 Saint Cyr sur Loire (FR)
(74) Mandataire: Thouret-Lemaitre, Elisabeth

(57) **Abrégé**

Poudres pour solutions buvables contenant du métoclopramide et du paracétamol ou un dérivé d'acide acétylsalicylique en association avec des excipients appropriés. Application en thérapeutique.

## Description

La présente invention a pour objet des compositions pharmaceutiques à base de métoclopramide et de p-acétamidophénol (paracétamol) ou de dérivés d'acide acétylsalicylique, sous forme de poudres pour solutions buvables.

Des compositions à base de métoclopramide et de paracétamol sous forme de poudres effervescentes et des compositions à base de métoclopramide et d'un agent analgésique tel que le paracétamol ou l'acide acétylsalicylique sous forme de comprimés, utiles dans le traitement de la migraine, sont décrites respectivement dans les brevets EP-0011489 et EP-0011490.

Les compositions de l'invention comprennent du métoclopramide ou l'un de ses sels, en particulier le monochlorhydrate monohydraté, et du paracétamol ou un dérivé d'acide acétylsalicylique qui est soit un ester, l'acétylsalicylate de p-acétamidophényle (acétylsalicylate de paracétamol), soit un sel choisi parmi l'acétylsalicylate d'arginine et les complexes constitués d'acétylsalicylate de sodium et de bicarbonate de sodium, d'acétylsalicylate de potassium et de bicarbonate de potassium, d'acétylsalicylate de calcium et d'urée, d'acétylsalicylate de magnésium et d'urée ou d'acide acétylsalicylique, de monochlorhydrate de L-lysine et de carbonate de calcium.

Ces compositions peuvent contenir d'autres constituants habituellement utilisés dans la formulation des poudres tels que des diluants, par exemple lactose, sorbitol, carbonate de calcium ou amidons, des substances tampons permettant d'ajuster le pH, par exemple citrates minéraux, carbonates et bicarbonates minéraux ou acides aminés, des édulcorants et des arômes naturels ou synthétiques.

Ces compositions sont utiles dans le traitement de la migraine.

Les poudres selon l'invention sont obtenues par mélanges successifs des différents constituants, par des procédés connus en soi. Elles peuvent ensuite être présentées sous forme de sachets dose unitaire.

Elles peuvent contenir l'équivalent de 5 à 20 mg de monochlorhydrate de métoclopramide et 250 à 1000 mg de paracétamol ou l'équivalent de 250 à 1000 mg d'acide acétylsalicylique par sachet dose unitaire.

Elles contiennent avantageusement l'équivalent de 10 mg de monochlorhydrate de métoclopramide et 450 mg de paracétamol ou l'équivalent de 900 mg d'acide acétylsalicylique par sachet dose unitaire.

Les formulations suivantes illustrent la présente invention.

### Exemple 1 :

| | |
|---|---|
| Complexe acétylsalicylate de sodium, bicarbonate de sodium | 1440,00 mg |
| Métoclopramide, monochlorhydrate monohydraté | 10,54 mg |
| Arôme + édulcorant | 49,46 mg |
| pour un sachet dose unitaire | 1500,00 mg |

### Exemple 2 :

| | |
|---|---|
| Complexe acétylsalicylate de potassium, bicarbonate de potassium | 1595,00 mg |
| Métoclopramide, monochlorhydrate monohydraté | 10,54 mg |
| Arôme + édulcorant | 44,46 mg |
| pour un sachet dose unitaire | 1650,00 mg |

### Exemple 3 :

| | |
|---|---|
| Complexe acétylsalicylate de calcium, urée | 1145,00 mg |
| Métoclopramide, monochlorhydrate monohydraté | 10,54 mg |
| Diluant | 194,46 mg |
| Tampon | 200,00 mg |
| Arôme + édulcorant | 50,00 mg |
| pour un sachet dose unitaire | 1600,00 mg |

### Exemple 4 :

| | |
|---|---|
| Complexe acétylsalicylate de magnésium, urée | 1106,00 mg |
| Métoclopramide, monochlorhydrate monohydraté | 10,54 mg |
| Diluant | 223,46 mg |
| Tampon | 210,00 mg |
| Arôme + édulcorant | 50,00 mg |
| pour un sachet dose unitaire | 1600,00 mg |

### Exemple 5 :

| | |
|---|---|
| Complexe acide acétylsalicylique, monochlorhydrate de L-lysine, carbonate de calcium | 2062,80 mg |
| Métoclopramide, monochlorhydrate monohydraté | 10,54 mg |
| arôme + édulcorant | 46,66 mg |
| pour un sachet dose unitaire | 2120,00 mg |

### Exemple 6 :

| | |
|---|---|
| Acétylsalicylate de paracétamol | 695,00 mg |
| Métoclopramide, monochlorhydrate monohydraté | 10,54 mg |
| Diluant | 244,46 mg |
| Arôme + édulcorant | 50,00 mg |
| pour un sachet dose unitaire | 1000,00 mg |

### Exemple 7 :

| | |
|---|---|
| Paracétamol | 450,00 mg |
| Métoclopramide, monochlorhydrate monohydraté | 10,54 mg |
| Diluant | 189,46 mg |
| Tampon | 200,00 mg |
| Arôme + édulcorant | 50,00 mg |
| pour un sachet dose unitaire | 900,00 mg |

La posologie journalière est de 1 à 3 sachets dose unitaire.

## Revendications

1. Poudre pour solution buvable caractérisée en ce qu'elle contient du métoclopramide ou l'un de ses sels et du paracétamol ou un dérivé d'acide acétylsalicylique choisi parmi l'acétylsalicylate de paracétamol, l'acétylsalicylate d'arginine et les sels complexes acétylsalicylate de sodium/bicarbonate de sodium, acétylsalicylate de potassium/bicarbonate de potassium, acétylsalicylate de calcium/urée, acétylsalicylate de magnésium/urée et acide acétylsalicylique/monochlorhydrate de L-lysine/carbonate de calcium, en association avec des excipients appropriés.

2. Poudre selon la revendication 1, caractérisée en ce que le métoclopramide est sous forme de monochlorhydrate monohydraté.

3. Poudre selon l'une quelconque des revendications 1 ou 2, caractérisée en ce qu'elle est présentée sous forme de sachets dose unitaire.

4. Poudre selon la revendication 3, caractérisée en ce qu'elle contient l'équivalent de 5 à 20 mg de monochlorhydrate de métoclopramide et 250 à 1000 mg de paracétamol ou l'équivalent de 250 à 1000 mg d'acide acétylsalicylique par sachet dose unitaire.

5. Poudre pour solution buvable selon la revendication 4, caractérisée en ce qu'elle contient l'équivalent de 10 mg de monochlorhydrate de métoclopramide et 450 mg de paracétamol ou l'équivalent de 900 mg d'acide acétylsalicylique par sachet dose unitaire.
